# EUROPEAN PATENT APPLICATION

(11) **EP 3 252 165 A1**
(43) Date of publication of application: **06.12.2017**
(21) Application number: 16305651.8
(22) Date of filing: 03.06.2016
(51) Int. Cl.: C12Q 1/68

(54) **METHOD FOR THE PROGNOSIS OF MULTIPLE MYELOMA**

(71) Applicant: Centre National de la Recherche Scientifique (C.N.R.S.), 75016 Paris (FR); UNIVERSITE DE MONTPELLIER, 34090 Montpellier (FR); Centre Hospitalier Universitaire de Montpellier, 34295 Montpellier (FR)
(72) Inventor: ALATERRE, Elina, 34570 MONTARNAUD (FR); MOREAUX, Jérôme, 34090 MONTPELLIER (FR)
(74) Representative: Nony

(57) **Abstract**

The present invention relates to the use of an expression level value of each of the four markers selected in a group consisting of CD24, CD27, CD36 and CD302 in a sample obtained from an individual affected with a multiple myeloma for predicting an outcome of a multiple myeloma in the said individual.

## Description

### FIELD OF THE INVENTION

The present invention relates to field of prognosis of multiple myeloma by measuring biological markers.

### BACKGROUND OF THE INVENTION

Multiple myeloma is one of the most common forms of hematological malignancy. It occurs in increasing frequently with advancing age, with a median age at diagnosis of about 65 years.

Multiple myeloma is the second most common hematological malignancy, although the exact causes of multiple myeloma remain unknown.

It is associated with an uncontrolled clonal proliferation of B-lymphocyte-derived plasma cells within the bone marrow. These myeloma cells replace the normal bone marrow cells, resulting in an abnormal production of cytokines, and in a variety of pathological effects, including for instance anemia, hypercalcemia, immunodeficiency, lytic bone lesions, and renal failure. The presence of a monoclonal immunoglobulin is frequently observed in the serum and/or urine of multiple myeloma patients.

Current treatments for multiple myeloma patients include chemotherapy, preferably associated when possible with autologous stem cell transplantation (ASCT).

Treatment of MM in 2015 (eligible for stem cell transplantation) consists of an induction phase with four monthly courses of high doses of corticosteroid (Dexamethasone) and a proteasome inhibitor, sometimes in association with a cell cycle targeting drug or an immunomodulatory drug, a short term exposure to high dose of melphalan (usually 200 mg per square meter), an alkylating agent followed with autologous stem cell transplantation to rescue hematopoiesis and a maintenance treatment with dexamethasone and an immunomodulatory drug. Approximately one-third of patients with myeloma at diagnosis are older than 75 years and at least 30% are frail, because of the presence of concomitant disease, abnormal laboratory test results and symptoms or signs of disability that may complicate the presentation and management of myeloma. Today, bortezomib-melphalan-prednisone (VMP) and melphalan-prednisone-thalidomide (MPT) are the reference treatments for elderly myeloma patients. Nevertheless, the efficacy of these regimens was less evident in patients aged 75 years or over. These findings raise the question of whether a lower dose intensity treatment with two-drug combinations may improve tolerability, preserving efficacy, in very elderly and frail patients, and thus should be preferred to three-drug combinations.

Survival of patients with multiple myeloma is highly heterogeneous from periods of few weeks to more than ten years. This variability derives from heterogeneity in both tumor and host factors.

Several factors correlated with survival duration in multiple myeloma have been identified, including in particular serum levels of hemoglobin, calcium, creatinine, B2- microglobulin (sβ2M), albumin, C-reactive protein (CRP), platelets count, proliferative activity of bone marrow plasma cells, and deletion of chromosome arm 13q. Subsequently, various combinations of prognostic factors have been suggested for staging classification of myeloma patients. A staging system for multiple myeloma has been developed through an international collaboration between several teams (GREIPP et al., J Clin Oncol, 23, 3412-20, 2005). A combination of serum β2M and serum albumin was retained as providing the simplest, most powerful and reproducible three-stage classification. This International Staging System (ISS) consists of the following stages: stage I, serum β2M less than 3.5 mg/L plus serum albumin >3.5 g/dL (median survival, 62 months); stage II, neither stage I nor III (median survival, 44 months); and stage III, Sβ2M >5.5 mg/L (median survival, 29 months).

It is thus important to identify factors associated with prognosis, in order to better predict disease outcome, and optimize patient treatment.

To diagnose and follow up minimal residual disease in MM, the expression of some markers have already been evaluated in the art by multiparametric flow cytometry (MFC) at protein level. CD19, CD20, CD27, CD28, CD56 and CD117 have been described in the art to discriminate BMPC from MMC (Rawstron et al., 2008, Haematologica, Vol. 91 : 1577-1578). The expression of these CD markers is not homogeneous on all MMC, on one hand all markers do not present aberrant expression and on the other hand expression level of each CD marker changes according to the sample (Gupta et al., 2009, Am. J Clin Pathol, Vol. 132 : 728-732; Raja et al., 2010, Br J Haematol, Vol. 149 : 344-351; Seegmiller et al., 2007, Am J Clin Pathol, Vol. 127 : 176-181; Tembhare et al., 2014, Leuk Res, Vol. 38(3): 371-376).

Indeed, MM is still an incurable disease even if some patients achieve a complete remission (CR). The treatment of relapsed/refractory patients is a challenge and the use of monoclonal therapeutic targets associated with others agents such as immunomodulatory drugs or proteasome inhibitor could be a new way to treat these patients. For example, anti-CD20 monoclonal antibodies (mAb) (rituximab, ofatumumab and obinutuzumab) were evaluated on patients and have improved event-free survival (EFS) and CR in chronic lymphocytic leukemia, another B-cell malignancy (Shah, 2015, Ther Clin Risk Manag,; Vol. 11 : 1113-1122). In MM, anti-CD40, CD38, CD138, CD56, B2-microglobulin, FGFR3 and CS-1 have demonstrated anti-tumor activity in preclinical models and in Phase I/II clinical trials (Donato et al., 2014, Expert Opin Bio Ther , Vol. 14(8): 1-18; van de Donk et al., 2012, Leukemia, Vol. 26 : 199-213). Moreover, the expression of some CD markers is known to be associated with prognosis value such as CD200 (Moreaux et al., 2006, Blood, Vol. 108 : 4194-4197).

Correlation between patient survival and novel markers would allow new prognosis factor identification and a better characterization of the disease including its classification in different stage of evolution. A better knowledge of the MM stage would permit to the clinicians to support patients with personalized treatment.

The invention has for purpose to meet the aforementioned needs.

### SUMMARY OF THE INVENTION

The present invention relates to the use of an expression level value of each of the four markers selected in a group consisting of CD24, CD27, CD36 and CD302 in a sample obtained from an individual affected with a multiple myeloma for predicting an outcome of a multiple myeloma in the said individual.

This invention also concerns *an in vitro* method for predicting outcome of multiple myeloma in an individual comprising the steps of:
a) measuring the expression level of each the four markers selected in a group consisting of CD24, CD27, CD36 and CD302 in a sample obtained from the said individual,
b) comparing the expression value measured at step a) with a reference value for each of the markers tested, and
c) determining the predicted outcome of multiple myeloma in the said individual from the comparison performed at step b).
In some embodiments of *the in vitro* prediction method, step c) comprises calculating a prognostic score value.

In some embodiments of *the in vitro* prediction method, the expression level of a marker selected in a group consisting of CD24, CD27, CD36 and CD302 consists of the level of gene expression of the said marker.

In some embodiments of *the in vitro* prediction method, the expression level of a marker selected in a group consisting of CD24, CD27, CD36 and CD302 consists of the level of protein expression of the said marker.

This invention also pertains to a method for determining the efficiency of a compound for treating a multiple myeloma in an individual comprising the steps of;
a) performing *the in vitro* method according to any one of claims 2 to 8 on a sample obtained from the said individual before treatment with the said compound, so as to determine a first prediction of outcome of a multiple myeloma in the said individual,
b) performing *the in vitro* method according to any one of claims 2 to 8 on a sample obtained from the said individual after treatment with the said compound, so as to determine a second prediction of outcome of a multiple myeloma in the said individual,
c) determining if the second prediction means a better outcome than the first prediction.

### DESCRIPTION OF THE FIGURES

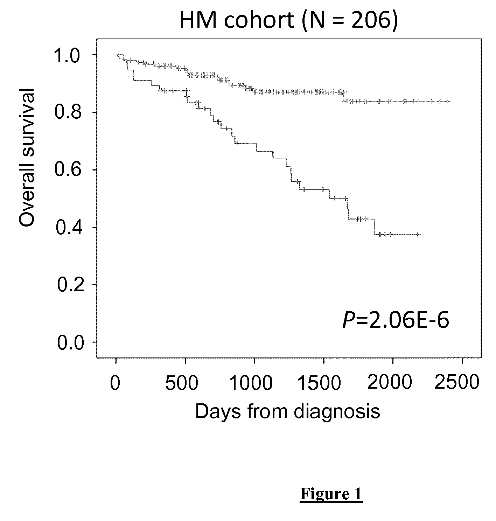

### Figure 1 : Prognostic value of CD Gene Risk Score (CDGRS) in multiple myeloma.

Using the Maxstat R function and Benjamini Hochberg multiple testing correction, 4 genes (CD24, CD27, CD36 and CD302) out of the 266 CD probesets were associated with a prognostic value in HM cohort (206 newly diagnosed patients). These 4 prognostic CD genes were used to create a CD gene risk score (CDGRS) resulting in two groups with different OS. Group 1 comprised 72.8% of patients with a high expression of all or 2 or 3 out of the 4 CD genes (upper curve) and group 2 comprised 27.2% of patients with a high expression of 1 out of 4 CD genes or a low expression of all CD genes (lower curve). Group 2 (lower curve) is associated with a significant shorter OS compared to group 1 (upper curve). Abscissa : days from diagnosis. Ordinate : Overall Survival (OS).

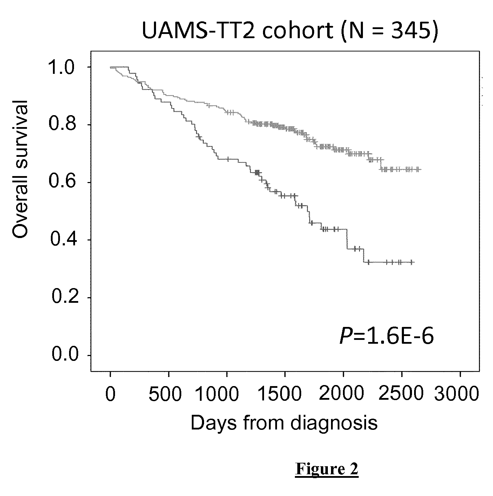

### Figure 2 : Prognostic value of CD Gene Risk Score (CDGRS) in multiple myeloma.

CDGRS was calculated in patients of UAMS-TT2 cohort (345 newly diagnosed patients) splitting patients in two groups. Group 1 comprised 72.8% of patients with a high expression of all or 2 or 3 out of the 4 CD genes (upper curve) and group 2 comprised 27.2% of patients with a high expression of 1 out of 4 CD genes or a low expression of all CD genes (lower curve). Group 2 (lower curve) is associated with a significant shorter OS compared to group 1 (upper curve). Abscissa : days from diagnosis. Ordinate : Overall Survival (OS).

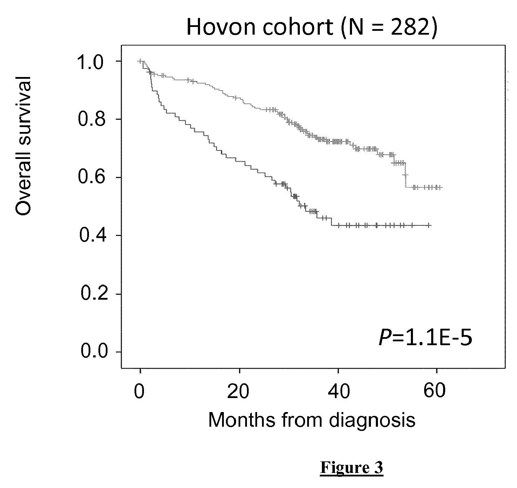

### Figure 3 : Prognostic value of CD Gene Risk Score (CDGRS) in multiple myeloma.

CDGRS was calculated in patients of Hovon cohort (282 newly diagnosed patients) splitting patients in two groups. Group 1 comprised 72.8% of patients with a high expression of all or 2 or 3 out of the 4 CD genes (upper curve) and group 2 comprised 27.2% of patients with a high expression of 1 out of 4 CD genes or a low expression of all CD genes (lower curve). Group 2 (lower curve) is associated with a significant shorter OS compared to group 1 (upper curve). Abscissa : days from diagnosis. Ordinate : Overall Survival (OS).

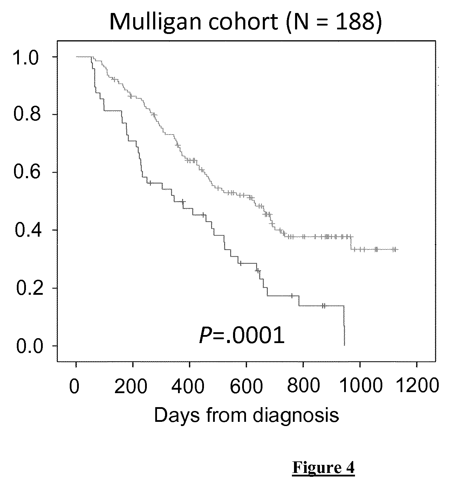

### Figure 4 : Prognostic value of CD Gene Risk Score (CDGRS) in multiple myeloma.

CDGRS was calculated in patients of Mulligan cohort (188 patients at relapse) splitting patients in two groups. Group 1 comprised 72.8% of patients with a high expression of all or 2 or 3 out of the 4 CD genes (upper curve) and group 2 comprised 27.2% of patients with a high expression of 1 out of 4 CD genes or a low expression of all CD genes (lower curve). Group 2 (lower curve) is associated with a significant shorter OS compared to group 1 (upper curve). Abscissa : days from diagnosis. Ordinate : Overall Survival (OS).

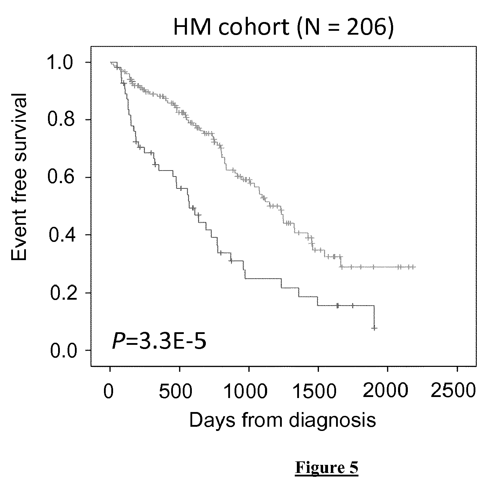

### Figure 5 : Prognostic value of CD Gene Risk Score (CDGRS) in multiple myeloma.

CDGRS was calculated in patients of HM cohort (206 newly diagnosed patients) splitting patients in two groups. Group 1 comprised 72.8% of patients with a high expression of all or 2 or 3 out of the 4 CD genes (upper curve) and group 2 comprised 27.2% of patients with a high expression of 1 out of 4 CD genes or a low expression of all CD genes (lower curve). Group 2 (lower curve) is also associated with a significant shorter EFS compared to group 1 (upper curve). Abscissa : days from diagnosis. Ordinate : Event Free Survival (EFS).

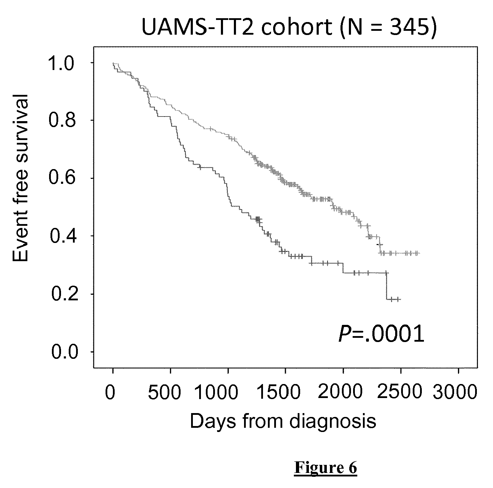

### Figure 6 : Prognostic value of CD Gene Risk Score (CDGRS) in multiple myeloma.

CDGRS was calculated in patients of UAMS-TT2 cohort (345 newly diagnosed patients) splitting patients in two groups. Group 1 comprised 72.8% of patients with a high expression of all or 2 or 3 out of the 4 CD genes (upper curve) and group 2 comprised 27.2% of patients with a high expression of 1 out of 4 CD genes or a low expression of all CD genes (lower curve). Group 2 (lower curve) is associated with a significant shorter EFS compared to group 1 (upper curve; Abscissa : days from diagnosis. Ordinate : Event Free Survival (EFS).

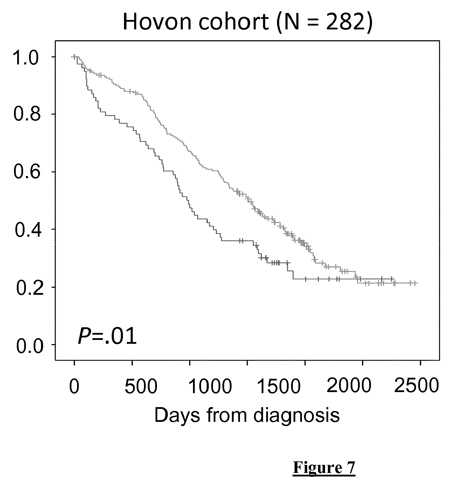

### Figure 7 : Prognostic value of CD Gene Risk Score (CDGRS) in multiple myeloma.

CDGRS was calculated in patients of Hovon cohort (282 newly diagnosed patients) splitting patients in two groups. Group 1 comprised 72.8% of patients with a high expression of all or 2 or 3 out of the 4 CD genes (upper curve) and group 2 comprised 27.2% of patients with a high expression of 1 out of 4 CD genes or a low expression of all CD genes (lower curve). Group 2 (lower curve) is associated with a significant shorter EFS compared to group 1 (upper curve). Abscissa : days from diagnosis. Ordinate : Event Free Survival (EFS).

### DETAILED DESCRIPTION OF THE INVENTION

The present invention provides for a novel prognosis marker of outcome of multiple myeloma in an individual.

The inventors have shown that a specific combination of biological markers is indicative of outcome in an individual with multiple myeloma.

More precisely, it is shown herein that the level of expression of a combination of markers selected in a group consisting of CD24, CD27, CD36 and CD302 is indicative of outcome of multiple myeloma in an individual.

The inventors have shown that the level of expression of a combination of markers selected in a group consisting of CD24, CD27, CD36 and CD302 allows predicting both (i) the overall survival time period (OS) of a patient affected with multiple myeloma and (ii) the time period free of a relapse event (EFS, also termed "Event Free Survival") of a patient affected with multiple myeloma.

The inventors have also shown that the level of expression of a combination of markers selected in a group consisting of CD24, CD27, CD36 and CD302 is indicative of the response of the said individual to a therapeutic treatment, e.g. a therapeutic treatment with the proteasome inhibitor such as bortezomib, mephalan, or a combination therapy such as a combination of dexamethasone and an immunomodulatory drug, a combination of bortezomib, mephalan and prednisone, a combination of mephalan, prednisone and thalidomide..

The level of expression of the combination of the markers CD24, CD27, CD36 and CD302 may be termed "CD-MM" marker herein.

As disclosed in the examples herein, the level of expression of each of the markers CD24, CD27, CD36 and CD302 may be used to define a score value that is indicative of the prognosis of multiple myeloma in a tested individual. As it is also shown herein, a relevant score value based on the expression level of each of the markers CD24, CD27, CD36 and CD302 may be determined according to various methods, which encompass calculating a CD Gene Risk Score (CDGR Score or CDGRS) with the Maxstat R function method (e.g. Maxstat software, release 0.7-23, Torsten Hotborn).

The inventors have further confirmed the relevancy of a prognosis score value based on a combination of the expression levels of each of the markers CD24, CD27, CD36 and CD302 by showing, through a gene set enrichment analysis, that patients having a low risk CDGR Score value exhibit a mature bone marrow plasma cell gene signature whereas patients having a high risk CDGR Score value exhibit a proliferation profile (undifferenciated plasmablasts) and MYC target genes related signature.

The present invention relates to the use of an expression level value of each of the four markers selected in a group consisting of CD24, CD27, CD36 and CD302 in a sample obtained from an individual affected with multiple myeloma for predicting outcome of multiple myeloma in the said individual.

This invention thus concerns the use of an expression level value of each of the four markers selected in a group consisting of CD24, CD27, CD36 and CD302 in a sample obtained from an individual affected with multiple myeloma for calculating a score value that is indicative of the prognosis of multiple myeloma in the said individual.

Using univariate COX and multivariate COX methods, it is shown in the examples herein that measuring the expression level of the combination of CD24, CD27, CD36 and CD302 consists of a specific marker that has been found to be independent from any of the other multiple myeloma other markers tested.

The CD24 gene encodes a sialoglycoprotein that is expressed on mature granulocytes and B cells and modulates growth and differentiation signals to these cells. The precursor protein is cleaved to a short 32 amino acid mature peptide which is anchored via a glycosyl phosphatidylinositol (GPI) link to the cell surface. The CD24 gene nucleic acid sequence may be retrieved (i) from the HGCN database under the accession number 1645, or (ii) from the Entrez_Gene database under the accession number Gene ID: 100133941. The CD24 amino acid sequence may be retrieved from the UniProt/SwissProt database under the accession number P25063.

The CD27 gene encodes a member of the TNF-receptor superfamily. This receptor is required for generation and long-term maintenance of T cell immunity. It binds to ligand CD70, and plays a key role in regulating B-cell activation and immunoglobulin synthesis. This receptor transduces signals that lead to the activation of NF-kappaB and MAPK8/JNK. The CD27 gene nucleic acid sequence may be retrieved (i) from the HGCN database under the accession number 11922, or (ii) from the Entrez-Gene database under the accession number Gene ID 939. The CD27 amino acid sequence may be retrieved from the UniProt/SwissProt database under the accession number P26842.

The CD36 gene encodes the fourth major glycoprotein of the platelet surface and serves as a receptor for thrombospondin in platelets and various cell lines. Since thrombospondins are widely distributed proteins involved in a variety of adhesive processes, this protein may have important functions as a cell adhesion molecule. It binds to collagen, thrombospondin, anionic phospholipids and oxidized LDL. The CD36 gene nucleic acid sequence may be retrieved (i) from the HGCN database under the accession number 1663, (ii) from the Entrez-Gene database under the accession number Gene ID 948. The CD36 amino acid sequence may be retrieved from the UniProt/SwissProt database under the accession number P16671.

The CD302 gene encodes a C-type lectin receptor involved in cell adhesion and migration, as well as endocytosis and phagocytosis. The CD302 gene nucleic acid sequence may be retrieved (i) from the HGCN database under the accession number 30843, (ii) from the Entrez-Gene database under the accession number Gene ID 9936. The CD302 amino acid sequence may be retrieved from the UniProt/SwissProt database under the accession number Q81X05.

The present invention also relates to *an in vitro* method for predicting outcome of multiple myeloma in an individual comprising the steps of:
a) measuring the expression level of each the four markers selected in a group consisting of CD24, CD27, CD36 and CD302 in a sample obtained from the said individual,
b) comparing the expression value measured at step a) with a reference value for each of the markers tested, and
c) determining the predicted outcome of multiple myeloma in the said individual from the comparison performed at step b).

In some embodiments of *the in vitro* prediction method, step c) comprises calculating a prognostic score value, as it will be detailed further in the present specification.

As used herein, the expression level of a marker encompasses both (i) the level of expression of the corresponding gene and (ii) the level of expression of the corresponding protein.

Thus, in some embodiments, the expression level of a marker selected in a group consisting of CD24, CD27, CD36 and CD302 consists of the level of expression of the said marker gene.

Also, in some embodiments, the expression level of a marker selected in a group consisting of CD24, CD27, CD36 and CD302 consists of the level of expression of the said marker protein.

It shall be understood that, for performing the prediction or prognosis method described herein, the expression levels which are measured are either (i) exclusively the gene expression levels of each of the markers selected in a group consisting of CD24, CD27, CD36 and CD302 or (ii) exclusively the protein expression levels of each of the markers selected in a group consisting of CD24, CD27, CD36 and CD302. Thus, the *in vitro* prediction method described herein does not encompass embodiments wherein the gene expression level is measured for some of the markers selected in a group consisting of CD24, CD27, CD36 and CD302 and wherein the protein expression level is measured for the remaining markers.

Within the scope of the invention, "an individual" is intended to mean any mammal, such as cat, dog, preferably a human being, irrespective of its age or gender. In particular, an individual encompassed by the invention is having multiple myeloma, and therefore the term "individual" also refers to an individual that may be undergoing or not a treatment against multiple myeloma, for example radiotherapy, chemotherapy and/or immunotherapy. In a preferred embodiment of the invention, an individual refers to any patient (preferably human) afflicted with multiple myeloma. The term "multiple myeloma" refers to multiple myeloma such as revised in the World Health Organisation Classification C90.

Within the scope of the invention, the term "sample" is intended to mean any biological sample derived from an individual, such as a fluid, including blood, saliva; a tissue; a cell sample, an organ; a biopsy. The term "sample" encompasses a bone marrow-derived sample and a blood-derived sample. In some embodiments, a biological sample refers to multiple myeloma cells, bone marrow or medullary cells.

The sample may be collected according to conventional techniques in the art, and used directly for diagnosis or alternatively stored for subsequent diagnosis. A tumour sample may be fresh, frozen or paraffin- embedded.

In the sense of the invention, "*comprising*" encompasses *"consisting of'* and *"consisting essentially of".*

In the sense of the invention, a "*reference value*" refers to the expected gene or protein expression level in the biological sample of an individual that is either (i) a control individual which is not affected with a multiple myeloma, or (ii) an individual for whom the occurrence of multiple myeloma is known or detectable. By extension, a reference value can be determined either from a single individual, or from a group of individuals.

Thus, the invention also relates to a method for screening compounds for treating, or reducing multiple myeloma in an individual, comprising at least a step of determining in a biological sample of said individual the gene or protein expression levels for each of the markers selected in a group consisting of CD24, CD27, CD36 and CD302.

In some embodiments, the level of gene or protein expression of a marker selected in a group consisting of CD24, CD27, CD36 and CD302 may be used as the sole multiple myeloma prognosis marker (i) in a method of prognosis of the outcome of multiple myeloma and (ii) in a method of prognosis of the responsiveness of a patient to a therapeutic treatment of multiple myeloma.

In some embodiments, the level of expression of the markers selected in the group consisting of CD24, CD27, CD36 and CD302 may be used in combination with one or more further multiple myeloma markers in a predicting method multiple myeloma individual outcome.

Within the scope of the invention, the expression "likelihood to efficiently respond to" is meant to intend that the individual having multiple myeloma may be subjected to stabilization, an alleviating, a curing or a reduction of the progression of the symptoms or the disease itself.

Within the scope of the present invention, the term "symptom" is intended to mean any noticeable response of the body to a multiple myeloma condition. In practice, a symptom encompasses high levels of proteins in the blood, serum, urine, and organs, including but not limited to M-protein and other immunoglobulins (antibodies), albumin, and β2 -microglobulin, as well as hyperviscosity, infections, anemia, hypercalcemia, immunodeficiency, lytic bone lesions, and renal failure, infections, signs and symptoms of light chain amyloidosis.

In some embodiments, individual having a multiple myeloma that efficiently respond to a treatment against multiple myeloma may be characterized by a significant reduction of the size of the tumor, a reduction of the invasive properties of the tumor cells, a reduction of the migration properties of the tumor cells, a reduction of the mean cancerous cells counts, a variation of known multiple myeloma biomarkers.

Each of these parameters may be evaluated accordingly to known methods routinely used in the art.

Within the scope of the invention, the expression "measuring the level of expression of a CD marker" is intended to mean quantitatively or semi-quantitatively measuring either the level of the corresponding CD protein expression or the level of the corresponding CD gene expression in the cells comprised within the sample. As used herein, a CD marker encompasses CD24, CD27, CD36 and CD302.

In practice, the measurement of the expression level of a CD protein may be performed accordingly to any known method in the art.

In some embodiments, said method may comprise a step of contacting the sample with a compound capable of selectively binding to the corresponding CD protein, said compound being subsequently detected. For example, a compound capable of selectively binding to a CD protein may be an antibody, such as, for example, a monoclonal antibody, or an aptamer.

In some embodiments, the level of a CD protein expression may be measured by using standard immunodiagnostic techniques, including immunoassays such as competition, direct reaction, or sandwich type assays. Such assays include, but are not limited to, agglutination tests; enzyme-labelled and mediated immunoassays, such as ELISAs; biotin/avidin type assays; radioimmunoassays; immunoelectrophoresis; immunoprecipitation; flow cytometry, immunohistochemical staining. In some embodiments, a compound capable of selectively binding to a CD protein may be immobilized onto a suitable solid support, prior to its use.

In some preferred embodiments, the level of a CD protein expression is measured by a flow cytometry method.

In embodiments wherein the expression level consists of the expression level of the said CD gene, among CD24, CD27, CD36 and CD302, any known RNA level measuring method may be used, and especially the Prime Flow® method of measuring the RNA level by flow cytometry may be used.

In embodiments wherein the expression level of a CD protein consists of the expression level of the said CD protein,, CD24, CD27, CD36 and CD302, any known flow cytometry method may be used, especially a flow cytometry method using a combination of antibodies, each antibody being specifically directed against each of the CD24, CD27, CD36 and CD302 proteins.

In some other embodiments, the level of CD protein expression may be measured by immunohistochemical staining, preferably by using a monoclonal antibody directed against the corresponding CD protein.

In some embodiments, monoclonal antibodies directed against a CD protein may be prepared accordingly to any method known from the state in the art, e.g. as described in Harlow and Lane ("Antibodies: A Laboratory Manual", 2nd Ed; Cold Spring Harbor Laboratory Press, Cold Spring Harbor, NY, 1988).

In practice, in order to perform immunohistochemistry, tissue sections may be obtained from a individual and fixed onto a glass slide by any suitable fixing agent, such as e.g. alcohol, acetone, and paraformaldehyde, to which is reacted an antibody. Conventional methods for immunohistochemistry are described in Harlow and Lane ("Antibodies A Laboratory Manual", Cold Spring Harbor Press, Cold Spring Harbor, New York, 1988), Ausubel et al. ("Current Protocols In Molecular Biology", John Wiley and Sons, NY, 1987) or Brauer et al. (FASEB J, 15, 2689-2701; 2001).

In some embodiments, commercially available monoclonal antibody directed against a CD protein may be suitable, such as :
- for CD24, clone ab64064 from abcam®, or clone ML5 from Biolegend®,
- for CD27, clone ab131254 from abcam®, or clone LG.3A10 from Biolegend®,
- for CD36, clone 17044 from abcam®, or clone 5-271 from Biolegend®, and
- for CD302, reference ABIN484932 from Antibodies_On_Line®, or clone SAB4300704from Sigma Aldrich®.

In some embodiments, the measure of the level of expression of a CD protein may be dependent of the intensity of the labelling obtained from the anti-CD protein antibody used and/or the number of cells expressing a noticeable amount of the said CD protein.

In practice, the measurement of the level of a CD gene expression may be performed accordingly to any known method in the art. As used herein, CD genes encompass CD24 gene, CD27 gene, CD36 gene and CD302 gene.

In some embodiments, the measurement of the level of a CD gene expression is performed by the measurement of the quantity of mRNA, notably by a method routinely used in the art. In practice, the total nucleic acids fraction contained in the sample may be obtained according to standard methods, such as using lytic enzymes or chemical solutions or extracted by nucleic-acid-binding resins following the manufacturer's instructions. The extracted mRNA may be subsequently detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

In preferred embodiments wherein the level of a CD gene expression is performed by the measurement of the quantity of mRNA, any known RNA level measuring method may be used, and especially the Prime Flow® method of measuring the RNA level by flow cytometry may be used.

In some embodiments, other methods of amplification including ligase chain reaction (LCR), transcription-mediated amplification (TMA), strand displacement amplification (SDA) and nucleic acid sequence based amplification (NASBA) may be used.

In practice, probes for hybridization and/or amplification may be designed and obtained by any known method in the art.

In some other embodiments, the measurement of the level of a CD gene expression is performed by the DNA chip analysis technology (Hoheisel, Nature Reviews, Genetics, 2006, 7:200-210). Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling.

Determination of the expression level of the genes can be performed by a variety of techniques. Generally, the expression level as determined is a relative expression level. More preferably, the determination comprises contacting the biological sample with selective reagents such as probes, primers or ligands, and thereby detecting the presence, or measuring the amount, of polypeptide or nucleic acids of interest originally in the biological sample. Contacting may be performed in any suitable device, such as a plate, microtiter dish, test tube, well, glass, column, and so forth. In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the biological sample. In a preferred embodiment, the expression level may be determined by determining the quantity of mRNA.

Methods for determining the quantity of mRNA are well known in the art. For example the nucleic acid contained in the biological sample is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Methods for determining the quantity of mRNA by RNA sequencing may also be used.

Other methods of amplification include ligase chain reaction (LCR), transcription-mediated amplification (TMA), strand displacement amplification (SDA) and nucleic acid sequence based amplification (NASBA).

Furthermore, CD24, CD27, CD36 and CD302 gene expression may be combined with flow cytometry approach using PrimeFlow® technology representing a powerful tool for risk stratification and outcome prediction in MM. The PrimeFlow® technology expands the typical flow cytometry application by allowing simultaneous detection of RNA transcripts and proteins in single cells. The assay, which is notably called the PrimeFlow™ RNA Assay, facilitates analysis of transcript and protein expression correlations as the cell changes over time or in response to stimulus. The assay employs a fluorescent in situ hybridization (FISH) technique for simultaneous detection of up to three RNA transcripts in a single cell using a standard flow cytometer. RNA detection may be teamed together with intracellular and cell surface antibody staining to elevate the understanding of single cell dynamics to a new dimension.

Illustratively, nucleic acid probes that hybridize with CD24 mRNA may be selected in group comprising RNAscope® 2.5 VS probe Ref SKU:313029 and RNAscope® 2.5 LS probe Ref SKU:313028 (Advanced Cell Diagnostics, Belgium).

Illustratively, nucleic acid probes that hybridize with CD27 mRNA may be selected in group comprising RNAscope® probe Ref SKU:415461 and RNAscope® 2.5 VS probe Ref SKU:415459 (Advanced Cell Diagnostics, Belgium).

Illustratively, nucleic acid probes that hybridize with CD36 mRNA comprise the CD36 probe Ref SKU:CD36-20-OR (Empire Genomics, Interchim, Montluçon, France).

Illustratively, nucleic acid probes that hybridize with CD302 mRNA comprise the CD302 probe Ref SKU:CD302-20-OR (Empire Genomics, Interchim, Montluçon, France).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from a biological samples and subjecting the RNAs to amplification and hybridization to specific probes, more particularly by means of a quantitative or semi-quantitative RT-PCR.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a biological sample from a test patient, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210)

In this context, the invention further provides a DNA chip comprising a solid support which carries nucleic acids that are specific to the CD24, CD27, CD36 and CD302 genes.

Within the scope of the invention, the expression "reference value" is intended to mean a value representing a "standard" level of expression of a selected CD gene, in conditions wherein the cells express the said CD gene at a physiological level.

In some embodiments, said reference value may be measured in a sample obtained from an individual who is not affected with a multiple myeloma ("multiple myeloma-free" individual), which encompasses a healthy individual, i.e. an individual without any medical condition or any history of medical condition.

In some embodiments, said reference value may represent a mean value measured in a plurality of samples obtained from multiple myeloma-free individual(s). In some embodiments, a plurality of samples encompasses at least 2, at least 3, at least 5, at least 10, at least 15, at least 25, at least 50, at least 100, at least 250, at least 500 samples. In some embodiments, one or more multiple myeloma-free individual(s) encompasses at least 2, at least 3, at least 5, at least 10, at least 15, at least 25, at least 50, at least 100, at least 250, at least 500 multiple myeloma-free individuals.

In some embodiments, the mean reference value may be gathered from a plurality of samples stocked and periodically revaluated.

In certain embodiments, said reference value may represent a mean value measured in a plurality of samples obtained from one or more individual(s) which are not affected with a multiple myeloma.

In some embodiments, a reference value may also represent a value representing a "standard" level of expression of a selected CD gene, from a sample obtained from an individual who is affected with multiple myeloma.

In some embodiments, the level of a CD protein expression in one sample may be measured, e.g. by immunohistochemical staining, and further analyzed by a semi-quantitative method comprising the scoring of said level of expression. In practice, cellular stains related to the combination of the selected CD24, CD27, CD36 and CD302 proteins in a sample may be computed and analyzed using a software, or visually analyzed, and a score may be attributed to said sample.

In some embodiments, the measured expression value of each CD marker may be classified as (i) a "high" expression value or (ii) a "low" expression value, respectively.

Regarding attribution of a score value, a 2 grades scoring may be attributed accordingly to the measured level of the selected CD24, CD27, CD36 and CD302 gene or protein expression, namely:
- a good outcome prognosis score when a high expression level of two or more markers selected in a group consisting of CD24, CD27, CD36 and CD302 is measured,
- a bad outcome prognosis score when a low expression level of three or more markers selected in a group consisting of CD24, CD27, CD36 and CD302 is measured.

Within the scope of the invention, the expression "good outcome" encompasses any amelioration of the medical condition, including stabilization, an alleviating, reduction of the progression of the symptoms or the disease itself, an extended survival time period, an extended relapse-free time period or a total remission.

Within the scope of the invention, the expression "good outcome" also encompasses a benefit in term of overall survival prognosis. In some embodiments, an overall survival prognosis benefit may account for a significant gain of life expectancy.

As also shown in the examples herein, a prognosis score corresponding to a good outcome may mean that the tested individual is at low risk and has a median event-free time period (EFS) and overall survival (OS) substantially longer than the median event-free time period (EFS) and OS of a tested individual having a prognosis score corresponding to a bad outcome.

As shown in the examples herein, the score which may be calculated from the expression level of each CD marker of the combination of the markers CD24, CD27, CD36 and CD302 is indicative of the predicted likelihood of said individual having a multiple myeloma to efficiently respond to a therapeutic treatment against multiple myeloma, which encompasses to efficiently respond to a therapeutic treatment with a proteasome inhibitor such as bortezomib.

In some embodiments, an individual having a multiple myeloma responding efficiently to a therapeutic treatment may be characterized by a better efficiency of the active ingredient(s) used according to the said therapeutic treatment against multiple myeloma. Therapeutic treatment of multiple myeloma encompasses chemotherapy, exposure to radiation, surgery, immunotherapy, stem cell transplant and plasmapheresis.

Many different types of drugs are used to treat multiple myeloma, which encompass Melphalan, Vincristine (Oncovin), Proteasome inhibitors, Cyclophosphamide (Cytoxan), Etoposide (VP-16), Doxorubicin (Adriamycin), Liposomal doxorubicin (Doxil) and Bendamustine (Treanda). Combinations of these drugs are more effective than any single drug. Often these drugs are combined with other types of drugs like corticosteroids, bisphosphonates, immunomodulating agents (drugs that will change the patient's immune response), antibodies or epigenetic agents.

Radiation therapy uses focused high-energy x-rays or particles that penetrate the tissues of the body to reach and destroy cancer cells. Radiation may be used to treat areas of bone damaged by myeloma that have not responded to chemotherapy and are causing pain. It's also the most common treatment for solitary plasmacytomas. The type of radiation therapy most often used to treat multiple myeloma or solitary plasmacytoma is called *external beam radiation therapy*.

Although surgery is sometimes used to remove single plasmacytomas, it's rarely used to treat multiple myeloma. When spinal cord compression causes paralysis, severe muscle weakness, or numbness, emergency surgery may be needed.

In a stem cell transplant, the patient gets high-dose chemotherapy (sometimes with radiation to the whole body) to kill the cells in the bone marrow (including the myeloma cells). Then the patient receives new, healthy blood-forming stem cells. When stem cell transplants were first developed, the new stem cells came from bone marrow, and so this was known as a *bone marrow transplant*. Now, stem cells are more often gathered from the blood (a peripheral blood stem cell transplant).

Stem cell transplant is commonly used to treat multiple myeloma. Before the transplant, drug treatment is used to reduce the number of myeloma cells in the patient's body. Stem cell transplants (SCT) encompass autologous stem cell transplants and allogeneic stem cell transplant.

In some embodiments, the outcome of multiple myeloma may be determined before a medical treatment intended to cure or reduce the disease. Said outcome may be determined within few days or few months prior initiating a therapeutic treatment intended to cure or reduce multiple myeloma.

In some embodiments, the outcome may be determined during the course of a medical treatment intended to cure or reduce multiple myeloma.

In some other embodiments, the outcome may be determined after a medical treatment intended to cure or reduce multiple myeloma. Said outcome may be determined within few days or few months after the end of the said therapeutic treatment.

In some embodiments, any determination of the outcome of an individual having a multiple myeloma, by measuring the expression level of the combination of CD24, CD27, CD36 and CD302 as disclosed herein, may be undertaken in order to adjust the medical treatment, which encompasses maintaining or stopping the treatment, increasing or decreasing the doses of the pharmaceutically active agent(s) to be administered, increasing or decreasing the time interval between two administrations of the pharmaceutically active agent(s).

In certain embodiments, the score that may be calculated form the values of the expression level of CD24, CD27, CD36 and CD302 as measured in step a) is indicative of a good outcome for said individual.

In a one aspect, the invention relates to a use for predicting a likelihood of an individual having a multiple myeloma to efficiently respond to a therapeutic treatment against multiple myeloma, which encompasses a therapeutic treatment with a proteasome inhibitor such as bortezomib.

As shown in the examples section below, the level of expression of the combination of CD24, CD27, CD36 and CD302 is significantly correlated with the efficacy of a therapeutic treatment of multiple myeloma.

In some embodiments, the level of expression of the combination of CD24, CD27, CD36 and CD302 for use as a biomarker may be combined to one or more other known biomarker(s) for predicting an outcome for an individual having a multiple myeloma or for predicting a likelihood of an individual having a multiple myeloma to efficiently respond to a therapeutic treatment against multiple myeloma.

In some embodiments, other biomarkers may be selected in a group comprising UAMS 70-gene (Blood, 2006, Vol. 109 : 2276-2284), IFM signature (J Clin Oncol, 2008, Vol. 26 : 4798-4805), Centrosome Index (Blood, 2008, Vol. 111 : 1603-1609), HZD cell death signature (Clin Cancer Res, 2010, Vol. 16 : 1856-1864), IL6-HMCL signature (Haematologica, 2011, Vol. 96 ; 574-582), RS score (Bioinformatics, 2013, Vol. 29 ; 1149-1157), Proliferation Index (Haematologica, 2011, Vol. 96 : 87-95), EMC 92 gene signature (Leukemia, 2012, Vol. 26 : 2406-2413) and Chromosome Instability genomic event count (CINGEC) signature (PLoS One, 2013, 8 : e66361).

In a one aspect, the invention relates to a use of a level of expression of the combination of CD24, CD27, CD36 and CD302 as a biomarker for determining a severity of multiple myeloma in an individual.

The simplest measure of prognosis in MM is based on blood levels of two markers: beta-2-microglobulin and albumin. In general, higher levels of beta-2-microglobulin and lower levels of albumin are associated with a poorer prognosis. This staging system is referred to as the International Staging System, or ISS.

A Revised International Staging System (R-ISS) has been developed which uses information about genetic changes in the myeloma cells to improve the prognostic value of the original ISS.

The Durie-Salmon staging system is an older system that divides patients into three stages: Stages I, II, and III, corresponding to low, intermediate, and high cell mass, depending upon the severity of anemia, calcium level, kidney function, presence or absence of bone lesions, and the quantity of abnormal proteins. This staging system is best used as a measure of the overall amounts of malignant plasma cells present in the patient, and is less useful as a measure of prognosis.

Risk stratification - The aggressiveness of MM depends upon several variables that impact disease biology. Genetic abnormalities seen in the myeloma cells are one of the strongest predictors of tumor aggressiveness. As such, all patients with newly diagnosed MM are classified as having high, intermediate, or standard risk disease based upon tumor genetics.

High risk disease - Approximately 15 percent of people with MM have high risk disease on cytogenetic testing. This includes patients with, for example, the following cytogenetic abnormalities: translocation t(14;16), translocation t (14;20) and deletion chromosome 17p. This type of MM is aggressive and may shorten survival. As such, patients with high risk disease are treated with more aggressive therapy.

Intermediate risk disease - Approximately 10 percent of people with MM have intermediate risk disease on cytogenetic testing. This includes patients with translocation t(4;14). This type of MM was previously considered to be high risk disease, but with appropriate therapy, patients with these findings have outcomes approaching that of standard risk MM.

Standard risk disease - All patients with MM who lack high or intermediate risk genetic abnormalities are considered to have standard risk MM. With modern therapy, patients with standard risk MM have an estimated median survival of 8 to 10 years.

In some embodiments of *the in vitro* prediction method described herein, the measured values of the expression level of the markers CD24, CD27, CD36 and CD302 are used to calculate a CD Gene Risk Score ("CDGR Score" or "CDGRS") by taking into account Expression Level Reference (ELRI)(calculated using Maxstat algorithm) for each of the CD24, CD27, CD36 and CD302 gene markers. These parameters are listed in Table 1 hereunder.

**Table 1 : list of the marker genes**

| **Gene Symbol** | **Gene ID** | **Reference level (ELRI)** |
|---|---|---|
| CD24 | 100133941 | ***296*** |
| CD27 | 939 | ***2055*** |
| CD36 | 948 | ***100*** |
| CD302 | 9936 | ***701*** |

### Embodiments of the method for predicting an outcome of a multiple myeloma

As already specified elsewhere in the present specification, the level of expression of each of the markers CD24, CD27, CD36 and CD302 may be used to define a score value that is indicative of the prognosis of multiple myeloma in a tested individual. As it is also shown herein, a relevant score value based on the expression level of each of the markers CD24, CD27, CD36 and CD302 may be determined according to various methods, which encompass calculating a CD Gene Risk Score (CDGR Score or CDGRS).

In some embodiments, the CDGR score value consists of a numerical value according to which a good outcome status or a bad outcome status of the tested individual may be determined.

In some other embodiments, the CDGR score value is directly the status of the tested patient, the said status being selected in the group consisting of (i) a good outcome status and (ii) a bad outcome status.

As it is detailed further in the present specification, a gene or protein expression value measured for each marker (i) may be labeled "High" if the measured expression value is higher than a reference value and (ii) may be labeled "Low" if the measured expression value is lower than a reference value. In some embodiments, the said reference value is a "cut-off" reference value, such as a score value which is termed (ELRi), the calculation of which is detailed further in the present specification.

According to these embodiments, a measured expression value that is higher than the reference value may be expressed as CD^{High} (encompassing CD24^{High}, CD27^{High}, CD36^{High} and CD302^{High}).

According to these embodiments, a measured expression value that is lower than the reference value may be expressed as CD^{Low} (encompassing CD24^{Low}, CD27^{Low}, CD36^{Low} and CD302^{Low}).

Thus, in some embodiments of *the in vitro* prediction method described herein, a good outcome status may be determined at the final step of this method for the following marker status :
- CD24^{High}, CD27^{High}, CD36^{Low} and CD302^{Low},
- CD24^{High}, CD27^{Low}, CD36^{High} and CD302^{High},
- CD24^{High}, CD27^{Low}, CD36^{High} and CD302^{Low},
- CD24^{High}, CD27^{Low}, CD36^{Low} and CD302^{High},
- CD24^{Low}, CD27^{High}, CD36^{High} and CD302^{Low},
- CD24^{Low}, CD27^{High}, CD36^{Low} and CD302^{High},
- CD24^{Low}, CD27^{Low}, CD36^{High} and CD302^{High},
- CD24^{High}, CD27^{High}, CD36^{High} and CD302^{Low},
- CD24^{High}, CD27^{High}, CD36^{Low} and CD302^{High},
- CD24^{Low}, CD27^{High}, CD36^{High} and CD302^{High}, and
- CD24^{High}, CD27^{High}, CD36^{High} and CD302^{High}.

Also, in some embodiments of *the in vitro* prediction method described herein, a bad outcome status may be determined at the final step of this method for the following marker status :
- CD24^{High}, CD27^{Low}, CD36^{Low} and CD302^{Low},
- CD24^{Low}, CD27^{High}, CD36^{Low} and CD302^{Low},
- CD24^{Low}, CD27^{Low}, CD36^{High} and CD302^{Low},
- CD24^{Low}, CD27^{Low}, CD36^{Low} and CD302^{High}, and
- CD24^{Low}, CD27^{Low}, CD36^{Low} and CD302^{Low}.

The present invention also relates to *an in vitro* method for predicting outcome of multiple myeloma in an individual comprising the steps of :
(a) determining the expression level (ELi) of of each the four markers selected in a group consisting of CD24, CD27, CD36 and CD302 in a sample obtained from the said individual,
(b) comparing the expression level (ELi) determined at step (a) with a predetermined reference level (ELRi)
(c) calculating a CDGR score trough the following criteria :
   Good outcome status:
      - CD24^{High}, CD27^{High}, CD36^{Low} and CD302^{Low},
      - CD24^{High}, CD27^{Low}, CD36^{High} and CD302^{High},
      - CD24^{High}, CD27^{Low}, CD36^{High} and CD302^{Low},
      - CD24^{High}, CD27^{Low}, CD36^{Low} and CD302^{High},
      - CD24^{Low}, CD27^{High}, CD36^{High} and CD302^{Low},
      - CD24^{Low}, CD27^{High}, CD36^{Low} and CD302^{High},
      - CD24^{Low}, CD27^{Low}, CD36^{High} and CD302^{High},
      - CD24^{High}, CD27^{High}, CD36^{High} and CD302^{Low},
      - CD24^{High}, CD27^{High}, CD36^{Low} and CD302^{High},
      - CD24^{Low}, CD27^{High}, CD36^{High} and CD302^{High}, and
      - CD24^{High}, CD27^{High}, CD36^{High} and CD302^{High}.
   Poor outcome status:
      - CD24^{High}, CD27^{Low}, CD36^{Low} and CD302^{Low},
      - CD24^{Low}, CD27^{High}, CD36^{Low} and CD302^{Low},
      - CD24^{Low}, CD27^{Low}, CD36^{High} and CD302^{Low},
      - CD24^{Low}, CD27^{Low}, CD36^{Low} and CD302^{High}, and
      - CD24^{Low}, CD27^{Low}, CD36^{Low} and CD302^{Low}.
(d) determining the predicted outcome of multiple myeloma in the said individual from the comparison performed at step (c).

The present invention further concerns *an in vitro* method for determining the efficiency of a compound for treating multiple myeloma in an individual comprising the steps of ;
a) performing *the in vitro* prediction method described herein on a sample obtained from the said individual before treatment with the said compound, so as to determine a first prediction of outcome of multiple myeloma in the said individual,
b) performing *the in vitro* prediction method described herein on a sample obtained from the said individual after treatment with the said compound, so as to determine a second prediction of outcome of multiple myeloma in the said individual,
c) determining if the second prediction means a better outcome than the first prediction.

The present invention also pertains to *an in vitro* method for determining the efficiency of a compound for treating multiple myeloma in an individual comprising the steps of ;
(a) determining the expression level (ELi) of of each the four markers selected in a group consisting of CD24, CD27, CD36 and CD302 in a sample obtained from the said individual,
(b) comparing the expression level (ELi) determined at step (a) with a predetermined reference level (ELRi)
(c) calculating a CDGR score trough the following criteria :
   Good response status:
      - CD24^{High}, CD27^{High}, CD36^{Low} and CD302^{Low},
      - CD24^{High}, CD27^{Low}, CD36^{High} and CD302^{High},
      - CD24^{High}, CD27^{Low}, CD36^{High} and CD302^{Low},
      - CD24^{High}, CD27^{Low}, CD36^{Low} and CD302^{High},
      - CD24^{Low}, CD27^{High}, CD36^{High} and CD302^{Low},
      - CD24^{Low}, CD27^{High}, CD36^{Low} and CD302^{High},
      - CD24^{Low}, CD27^{Low}, CD36^{High} and CD302^{High},
      - CD24^{High}, CD27^{High}, CD36^{High} and CD302^{Low},
      - CD24^{High}, CD27^{High}, CD36^{Low} and CD302^{High},
      - CD24^{Low}, CD27^{High}, CD36^{High} and CD302^{High}, and
      - CD24^{High}, CD27^{High}, CD36^{High} and CD302^{High}.
   Poor response status:
      - CD24^{High}, CD27^{Low}, CD36^{Low} and CD302^{Low},
      - CD24^{Low}, CD27^{High}, CD36^{Low} and CD302^{Low},
      - CD24^{Low}, CD27^{Low}, CD36^{High} and CD302^{Low},
      - CD24^{Low}, CD27^{Low}, CD36^{Low} and CD302^{High}, and
      - CD24^{Low}, CD27^{Low}, CD36^{Low} and CD302^{Low}.
(d) determining the predicted response status of multiple myeloma in the said individual from the comparison performed at step (c).

In some embodiments, the said therapeutic treatment comprises a treatment with a proteasome inhibitor such as bortezomib, mephalan, or with a combination therapy such as with a combination of dexamethasone and an immunomodulatory drug, a combination of bortezomib, mephalan and prednisone, a combination of mephalan, prednisone and thalidomide, as illustrative embodiments.

Predetermined reference values ELRi used for comparison may consist of "cut-off" values.

For example; each reference ("cut-off") value ELRi for each gene selected in a group consisting of the CD24 gene, the CD27 gene, the CD36 gene and the CD302 gene may be determined by carrying out a method comprising the steps of:
a) providing a collection of samples from patients suffering of multiple myeloma;
b) determining the expression level of the relevant gene for each sample contained in the collection provided at step a);
c) ranking the samples according to said expression level
d) classifying said samples in pairs of subsets of increasing, respectively decreasing, number of members ranked according to their expression level,
e) providing, for each sample provided at step a), information relating to the actual clinical outcome for the corresponding cancer patient (i.e. the duration of the event-free survival (EFS) or the overall survival (OS) or both);
f) for each pair of subsets of tumour tissue samples, obtaining a Kaplan Meier percentage of survival curve;
g) for each pair of subsets of tumour tissue samples calculating the statistical significance (p value) between both subsets
h) selecting as reference value ELR for the expression level, the value of expression level for which the p value is the smallest.

For example, the expression level of a gene Gi selected in the group consisting of CD24 gene, CD27 gene, CD36 gene and CD302 gene has been assessed for 100 samples of 100 patients. The 100 samples are ranked according to the expression level of gene Gi. Sample 1 has the highest expression level and sample 100 has the lowest expression level. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding cancer patient, Kaplan Meier curves are prepared for each of the 99 groups of two subsets. Also for each of the 99 groups, the p value between both subsets was calculated. The reference value ELRi is then selected such as the discrimination based on the criterion of the minimum p value is the strongest. In other terms, the expression level corresponding to the boundary between both subsets for which the p value is minimum is considered as the reference value. It should be noted that according to the experiments made by the inventors, the reference value ELRi is not necessarily the median value of expression levels.

In some embodiments, the reference value ELRi for the genes are described in **Table 1** (right column).

The invention also relates to a kit for performing the methods as above described, wherein said kit comprises means for measuring the expression level of the genes or proteins selected in the group consisting of CD24, CD27, CD36 and CD302. Typically the kit may include a primer, a set of primers, a probe, a set of probes, a method of sequencing, as a set of antibodies above described. In a particular embodiment, the probe or set of probes are labelled as above described. The kit may also contain other suitably packaged reagents and materials needed for the particular detection protocol, including solid-phase matrices, if applicable, and standards.

In some embodiments, the CDGR score may be generated by a computer program.

### Methods of treatment

The method of the invention allows to define a subgroup of patients who will be responsive ("responder") or not responsive ("non responder") to a treatment with a therapeutic compound, which encompasses a proteasome inhibitor such as bortezomib, mephalan, or with a combination therapy such as with a combination of dexamethasone and an immunomodulatory drug, a combination of bortezomib, mephalan and prednisone, a combination of mephalan, prednisone and thalidomide, as illustrative embodiments.

A further object of the invention relates to a method for the treatment of multiple myeloma in a patient in need thereof.

In the context of the invention, the term "treating" or "treatment", as used herein, means reversing, alleviating, inhibiting the progress of, or preventing the disorder or condition to which such term applies, or one or more symptoms of such disorder or condition.

In some embodiments, the method comprises the following steps
a) testing whether the patient will respond or not to a therapeutic compound by performing *the in vitro* prediction method described herein,
b) administering the said therapeutic compound, if said patient has as a CDGR score related to high risk (i.e. the patient will respond to the said therapeutic compound).

In some embodiments, the said therapeutic compound is a proteasome inhibitor such as bortezomib.

A further object of the invention relates to a therapeutic compound for use in the treatment of multiple myeloma in a patient in need thereof, wherein the patient was being classified as responder to the said therapeutic compound by *the in vitro* prediction method disclosed herein.

The invention will be further illustrated, without being limited to, by the following figures and examples.

### EXAMPLES

### A. MATERIAL AND METHODS

### A.1. Patient samples and gene expression data

Data of two independent cohorts of previously untreated MM patients was used. The training cohort consists of 206 MM patients and is termed the Heidelberg-Montpellier (HM)-cohort. The samples were obtained after written informed consent in accordance with the Declaration of Helsinki and after approval of the ethics committee of Montpellier and Heidelberg. These data are publicly available through ArrayExpress database (E-MTAB-372). The validation cohort contains 345 MM patients from the University of Arkansas for Medical Sciences (UAMS, Little Rock, AR, USA) and is referred as TT2-cohort. These data can be accessed at the online Gene Expression Omnibus (GSE2658). The clinical characteristics of the cohorts have been previously described (Moreaux et al., 2012, Mol Cancer Ther.; 11(12):2685-92 Hose et al, Haematologica., 2011 Jan;96(1):87-95.). GEP data from a cohort of 282 patients included in the Dutch-Belgian/German HOVON-65/ GMMG-HD4 trial have been used (accession number GSE19784). The clinical characteristics of this cohort have been previously described (Leukemia., 2012 Nov;26(11):2406-13). Affymetrix data of 152 relapsed MM patients subsequently treated with bortezomib (GSE9782) from the study by Mulligan *et* al. were also used.

### A.2. Gene expression profiling and statistical analyses

Gene expression data were analysed with SAM (Significance Analysis of Microarrays). Probe sets were selected for prognostic significance using Maxstat R function and Benjamini Hochberg multiple testing correction. The statistical significance of differences in overall survival (OS) and EFS between groups of patients was calculated by the log-rank test. Multivariate analysis was performed using the Cox proportional hazards model. Survival curves were plotted using the Kaplan-Meier method. All these analyses have been done with R.2.10.1 and Bioconductor version 2.5.

### A.3. Gene Set Enrichment Analysis (GSEA)

The gene expression levels from high risk CD-score versus low risk CD-score MM patients were compared and the genes which had significant different expression for GSEA were picked up. Gene set enrichment analysis was carried out by computing overlaps with canonical pathways and gene ontology gene sets obtained from the broad institute.

### B. RESULTS

### Example 1 : Prognostic value of CD genes in multiple myeloma and building of a CD gene risk score

Using the Maxstat R function and Benjamini Hochberg multiple testing correction, 4 genes (CD24, CD27, CD36 and CD302) were associated with a prognostic value in two independent cohorts of patients (HM cohort, n=206 and UAMS TT2 cohort, n=345). A high expression of CD24, CD27, CD36 and CD302 is associated with a good prognosis.

These 4 prognostic CD genes were used to create a CD gene risk score. To combine the prognostic information of CD24, CD27, CD36 and CD302, a simple staging scoring was built splitting patients in 5 groups according to CD24, CD27, CD36 and CD302 expression. Group 1 comprised patients with CD24, CD27, CD36 and CD302 low expression, group 2 included patients with high expression of 1 out of the 4 genes. The group 3 consisted of patients with high expression of 2 out of the 4 genes, group 4 comprised patients with high expression of 3 out of the 4 genes and the group 5 consisted of patients with a high expression of CD24, CD27, CD36 and CD302. Kaplan Meier analysis of the 5 different groups was performed. When 2 consecutive groups shown no significant difference, they were merged. This approach resulted in two groups with different OS. Group 1 comprised 72.8% of patients with a high expression of all or 2 or 3 out of the 4 CD genes and group 2 comprised 27.2% of patients with a high expression of 1 out of 4 CD genes or a low expression of all CD genes (Figure 1). Group 1 (low risk) had a not reached median OS and group 2 (high risk) had a median OS of 50.6 months *(P =* 2.06E-6) (Figure 1).

The prognostic value of the CD gene risk score was validated in two independent cohorts of patient (LR-TT2 cohort; n=345 patients and Hovon cohort n=282)) with a not reached median OS for group 1 and a 55.5 months median OS for group 2 (*P* = 1.6E-6) in LR-TT2 cohort and a 33.4 months median OS for group 2 in Hovon cohort (*P* = 1.1E-5) (Figures 2 and 3).

The CD-score could also predict for EFS in the HM cohort (P = 3.3E-5)(Figure 5). This prognostic value was validated in the LR-TT2 cohort (P = .0001) and the Hovon cohort (P = .01) (Figures 6 and 7).

We investigated if the CD-score could have a prognostic value for patients at relapse. Using a cohort of relapsing patients treated by bortezomib, the CD-score remained a prognostic factor separating patients in two groups with significantly different OS (median OS of 11.3 months for the high-risk group and 23.8 months for the low-risk group; P = 0.0001) (Figure 4).

### Example 2 : COX univariate and multivariate analyses of CD gene risk score

The CD-score prognostic value was compared with conventional prognostic factors and other gene-based risk score. In univariate COX analysis, CD-score, ISS (Greipp et al., 2005, J Clin Oncol, Vol. 23 : 3412-3420), β2M, Albumin, t(4;14), del17p, HRS (Shaughnessy et al., 2007, Blood, Vol. 109 : 2276-2284), IFM (Decaux et al., 2008, J Clon Oncol, Vol. 26 : 4798-4805), GPI (Hose et al., 2011, Haematologica, Vol. 69 : 87-95) and RS (Rème et al., 2013, Bioinformatics, Vol. 29 : 1149-1157) have prognostic value (Table 2A).

When analysed 2 by 2, CD-score tested with ISS, b2M, t(4; 14), HRS, GPI and RS remained independent in the HM cohort (Table 3). In the LR-TT2 cohort, CD-score, remained significant when tested with t(4;14), HRS, IFM score, GPI and RS (Table 2B).

When all parameters were tested together, CD-score, b2M, t(4; 14), IFM score and RS score remained significant in the HM cohort. In the LR-TT2 cohort, CD-score, t(4; 14) and HRS remained independent prognostic factors (Table 2C).

### Table 2: Cox univariate and multivariate analysis of overall survival (OS) in HM and TT2 patients' cohorts.

**Table 2A**

| **Univariate COX analysis** | | **HM-cohort** | | **TT2-cohort** | |
|---|---|---|---|---|---|
| **Overall Survival** | | **OS** | | **OS** | |
| | Pronostic variable | Proportional hazard ratio | P-value | Proportional hazard ratio | P-value |
| | CD-score | 0,25 | <0.0001 | 0,49 | <0.0001 |
| | ISS | 1,84 | 0,002 | NA | NA |
| | B2m | 1,1 | <0.0001 | NA | NA |
| | t(4 ;14) | 3,32 | <0.0001 | 2,21 | 0,001 |
| | del17p | 3,44 | 0,02 | NA | NA |
| | HRS | 2,37 | 0,01 | 4,67 | <0.0001 |
| | IFM score | 2,49 | 0,01 | 1,78 | 0,004 |
| | GPI | 2,54 | <0.0001 | 1,75 | <0.0001 |
| | RS | 4,16 | <0.0001 | 1,91 | <0.0001 |

**Table 2B**

| **2 by 2 Multivariate COX analysis** | | **HM-cohort** | | **TT2-cohort** | |
|---|---|---|---|---|---|
| **Overall Survival** | | **OS** | | **OS** | |
| | Pronostic variable | Proportional hazard ratio | *P*-value | Proportional hazard ratio | *P*-value |
| | CD-score | 0,27 | <0.0001 | NA | NA |
| | ISS | 1,75 | 0,008 | NA | NA |
| | CD-score | 0,27 | <0.0001 | NA | NA |
| | B2m | 1,1 | 0,002 | NA | NA |
| | CD-score | 0,25 | <0.0001 | 0,49 | <0.0001 |
| | t(4 ;14) | 3,12 | 0,001 | 2,25 | <0.0001 |
| | CD-score | 0,24 | <0.0001 | NA | NA |
| | del17p | 2,51 | NS | NA | NA |
| | CD-score | 0,21 | <0.0001 | 0,63 | 0,006 |
| | HRS | 3,11 | 0,002 | 3,68 | <0.0001 |
| | CD-score | 0,26 | <0.0001 | 0,43 | <0.0001 |
| | IFM score | 1,89 | NS | 1,58 | 0,04 |
| | CD-score | 0,28 | <0.0001 | 0,52 | <0.0001 |
| | GPI | 2,29 | 0,001 | 1,5 | 0,009 |
| | CD-score | 0,37 | 0,002 | 0,56 | <0.0001 |
| | RS | 3,42 | <0.0001 | 1,68 | <0.0001 |

**Table 2C**

| Multivariate COX analysis | | HM-cohort | | TT2-cohort | |
|---|---|---|---|---|---|
| Overall Survival | | OS | | OS | |
| | Pronostic variable | Proportional hazard ratio | *P-*value | Proportional hazard ratio | P-value |
| | CD-score | 0,27 | 0,001 | 0,63 | 0,007 |
| | ISS | 1,38 | NS | NA | NA |
| | B2m | 1,1 | 0,03 | NA | NA |
| | t(4 ;14) | 3,01 | 0,01 | 2,07 | 0,003 |
| | del17p | 1,26 | NS | NA | NA |
| | HRS | 2,04 | NS | 2,93 | <0.0001 |
| | IFM score | 0,28 | 0,03 | 0,95 | NS |
| | GPI | 1,47 | NS | 1,18 | NS |
| | RS | 2,48 | 0,02 | 1,1 | NS |

### Example 3 : MMC of patients in low-risk CD-score group have a mature bone marrow plasma cell gene signature whereas MMC from patients in high risk CD-score group are characterized by proliferation and MYC target genes related signature.

Gene set enrichment analysis was performed to compare gene expression profiles of patient's MMC in high- and low-risk CD-score groups (n=71 and n=135 respectively within the HM cohort).

Genes related to mature bone marrow plasma cells (P=0.003) and to normal bone marrow plasma cells (P=0.01) were significantly enriched in low-risk CD-score group ().

Patients of the high-risk CD-score group were characterized by a significant enrichment of genes related to undifferentiated plasma blasts (*P*<0.0001), MYC target genes (*P*=0.01) and myeloma (*P*=0.01).

Altogether, the experimental data disclosed herein report a CD gene based risk score (including CD27, CD24, CD302 and CD36) with a prognostic value in three independent cohorts of patients. Patients in low-risk CD score group have a significant enrichment of genes related to mature BPMC microenvironment-dependent. However, high-risk CD-score patient group overexpressed genes associated with undifferentiated cancer cells.

In high-risk CD-score patient group is also associated with pathways related to TFRC and MYC genes. These two genes are known to be involved in proliferation of MMC. TFRC (CD71), the transferrin receptor, is involved in cellular iron uptake and cell growth. CD71 is expressed at low level in normal cells and is upregulated in proliferative cells (Loisel et al., 2011), its expression can be correlated to disease progression (Daniels et al., 2012). Anti-CD71 conjugated with avidin showed antitumor activity and long-term survival in MM xenograft mouse models (Daniels et al., 2011). MYC is a proto-oncogene dysregulated or overexpressed in many cancers particularly in MM where it has a prominent role in pathogenesis. MYC encodes a transcriptional factor that regulates cell proliferation, growth, protein translation, metabolism and apoptosis. MYC rearrangements are observed in 15% of MM patients at diagnosis and relapse. Upregulation of MYC is associated with disease progression, evolution from MGUS to MM and poor prognosis (Glitza et al., 2015, Leuk Lymphoma, Vol. 56 : 602-607).

## Claims

1. The use of an expression level value of each of the four markers selected in a group consisting of CD24, CD27, CD36 and CD302 in a sample obtained from an individual affected with multiple myeloma for predicting outcome of multiple myeloma in the said individual.

2. An *in vitro* method for predicting outcome of multiple myeloma in an individual comprising the steps of :
a) measuring the expression level of each the four markers selected in a group consisting of CD24, CD27, CD36 and CD302 in a sample obtained from the said individual,
b) comparing the expression value measured at step a) with a reference value for each of the markers tested, and
c) determining the predicted outcome of multiple myeloma in the said individual from the comparison performed at step b).

3. *The in vitro* method according to claim 2, wherein step c) comprises calculating a prognostic score value.

4. *The in vitro* method according to any one of claims 2 and 3, wherein the expression level of a marker selected in a group consisting of CD24, CD27, CD36 and CD302 consists of the level of gene expression of the said marker.

5. *The in vitro* method according to any one of claims 2 and 3, wherein the expression level of a marker selected in a group consisting of CD24, CD27, CD36 and CD302 consists of the level of protein expression of the said marker.

6. *The in vitro* method according to any one of claims 1 to 5, wherein a high expression level of two or more markers selected in a group consisting of CD24, CD27, CD36 and CD302 is indicative of a good outcome of multiple myeloma in the said individual.

7. *The in vitro* method according to any one of claims 1 to 5, wherein a low expression level of three or more markers selected in a group consisting of CD24, CD27, CD36 and CD302 is indicative of a bad outcome of multiple myeloma in the said individual.

8. *The in vitro* method according to any one of claims 1 to 7, wherein the said sample is selected in a group comprising a bone marrow-derived sample and a blood-derived sample.

9. A method for determining the efficiency of a compound for treating a multiple myeloma in an individual comprising the steps of;
a) performing *the in vitro* method according to any one of claims 2 to 8 on a sample obtained from the said individual before treatment with the said compound, so as to determine a first prediction of outcome of multiple myeloma in the said individual,
b) performing *the in vitro* method according to any one of claims 2 to 8 on a sample obtained from the said individual after treatment with the said compound, so as to determine a second prediction of outcome of multiple myeloma in the said individual,
c) determining if the second prediction means a better outcome than the first prediction.
